# EUROPEAN PATENT SPECIFICATION

(11) **EP 4 364 645 B1**
(45) Date of publication and mention of the grant of the patent: **07.01.2026**
(21) Application number: 22205349.8
(22) Date of filing: 03.11.2022
(51) Int. Cl.: A61B 5/00

(54) **METHOD AND SYSTEM FOR MONITORING VITAL DATA OF AN OCCUPANT OF A VEHCILE**
VERFAHREN UND SYSTEM ZUR ÜBERWACHUNG VON VITALDATEN EINES FAHRZEUGINSASSEN
PROCÉDÉ ET SYSTÈME DE SURVEILLANCE DE DONNÉES VITALES D'UN OCCUPANT D'UN VÉHICULE

(43) Date of publication of application: 08.05.2024
(73) Proprietor: Volvo Car Corporation, 405 31 Göteborg (SE)
(72) Inventor: LIND, Niclas, 40531 Göteborg (SE); SWÄRD, Billy, 40531 Göteborg (SE); MAGNUSSON, Klara, 40531 Göteborg (SE); VINNÅ, Bure Råman, 40531 Göteborg (SE)
(74) Representative: Maiwald GmbH

(56) References cited:
- US-A1- 2013 338 465
- US-A1- 2014 106 726
- US-A1- 2016 066 127
- US-A1- 2017 020 444
- US-A1- 2019 061 772
- US-A1- 2022 047 215
- US-A1- 2022 121 774

## Description

### TECHNICAL FIELD

The present disclosure relates to a method for monitoring vital data of an occupant of a vehicle, and a system for monitoring vital data of an occupant in a vehicle.

### BACKGROUND ART

Nowadays, vehicles comprise several systems for monitoring a driver and/or other passengers in the vehicle to increase safety while driving the vehicle. For example, there are known systems for reducing the risk of (heavy) crashes/accidents, particularly improving a dynamic driving behavior of the vehicle, such as ABS, ESP etc., systems for reducing an injury level of passengers in the vehicle, particularly in case of crashes/accidents such as seat belts, airbags as well as systems for supporting the driver while driving, such as detecting whether the driver is tired, lane departure warning systems (LDWS), i.e., lane keeping assist (LKA) and lane centering assist (LCA), collision avoidance systems (CAS) etc. Further, portable medical devices, such as a blood sugar meter, a blood pressure monitor, an oximeter, a hear rate monitor etc., as well as non-medical devices, such as smart watches, fitness tracker, smartphones etc. for monitoring at least one vital sign of a person wearing/using such a device are more and more common and popular, respectively.

US 2016/066127 A1 discloses a method for controlling and an electronic device thereof. A control method and an electronic device thereof are provided. A first electronic device can include a communication unit and a control unit configured to perform providing device identification information to a second electronic device based on a beacon received from the second electronic device through the communication unit, and conducting at least one function corresponding to the signal received from the second electronic device.

US 2019/061772 A1 relates to a system and method for monitoring the state of health and/or the well-being of a vehicle occupant. The system comprises a control unit, which comprises the following: a receiver for the wireless reception of physiological parameters of at least one unit which is worn on the body, which comprises one or more sensors for determining one or more physiological parameters of the vehicle occupant, and a diagnostic module which is designed to derive information regarding the state of health, the state of well-being, or of illnesses, at least partially on the basis of the physiological parameters received. The control unit is also designed to provide the vehicle occupants with information regarding the state of health by means of at least one output unit of the vehicle and to initiate at least one of the following steps: adapt vehicle functions to the state, or, by means of at least one output unit of the vehicle, to suggest or interactively to carry out measures that improve the state.

US 2014/106726 A1 shows a system and method for monitoring apps in a vehicle to reduce driver distraction. A controller operating inside or in combination with the head unit of the vehicle may monitor operation of the vehicle, and generate alerts indicative of operation of the vehicle (such as an alert indicative that the vehicle is operating at a predetermined speed). In response to the alert, the operation of the app in the vehicle may be modified.

US 2013/338465 A1 discloses displays for a medical device. Embodiments described herein relate to an analyte monitoring device having a user interface with a display and a plurality of actuators. The display is configured to render a plurality of display screens, including a home screen and an alert screen. The home screen is divided into a plurality of simultaneously displayed panels, with a first panel displays a rate of change of continuously monitored analyte levels in interstitial fluid, a second panel simultaneously displays a current analyte level and an analyte trend indicator, and a third panel displays status information of a plurality of components of the device. When an alarm condition is detected, the display renders the alert screen in place of the home screen, the alert screen displaying information corresponding to the detected alarm condition. Furthermore, the actuators are configured to affect further output of the analyte monitoring device corresponding to the detected condition.

US 2022/047215 A1 relates to an electronic device and method for measuring a vital signal. Methods and apparatuses are provided for measuring a biometric signal by an electronic device. An object having an animation effect for inhalation and exhalation is displayed. The animation effect includes the object being expanded to guide the inhalation of a user and the object being contracted to guide exhalation of the user. A sensor unit obtains at least one biometric signal related to a heart rate of the user while the object is displayed. A motion of the electronic device is detected having a strength that is greater than or equal to a threshold strength. Based on the motion ceasing before exceeding a predetermined time, display of the object and obtaining of the at least one biometric signal is continued. Based on the motion lasting longer than or equal to the predetermined time, the obtaining of the at least one biometric signal and the displaying of the object is terminated.

### SUMMARY

There may, therefore, be a need to provide an improved method and/or system for monitoring vital data of an occupant in a vehicle, which may increase an awareness of vital signs of the occupant, particularly the driver, facilitate the occupant to detect critical vital signs, and particularly may support the occupant in reacting to detected critical vital signs accordingly.

The object of the present disclosure is at least partially solved or alleviated by the subject-matter of the appended independent claims, wherein further examples are incorporated in the dependent claims.

According to a first aspect, there is provided a method for monitoring vital data of an occupant, particularly a driver, of a vehicle. The method comprises:
receiving, by an on-board-device of the vehicle, personal data comprising at least one vital sign from the personal device from the occupant, particularly via a data transmitting connection, the personal device being configured to collect and/or record the personal data comprising the at least one vital sign;
storing the received personal data in a storing unit,
setting at least one of a predetermined upper threshold and a predetermined lower threshold for the at least one vital sign;
displaying at least a part of the stored personal data on a display device, the predetermined upper threshold and/or the predetermined lower threshold for the at least one vital sign, wherein the part of the stored personal data comprises at least a current value of the at least one vital sign;
comparing the current value of the at least one vital sign with the predetermined upper threshold and/or the predetermined lower threshold;
notifying the occupant visually and/or audibly and/or physically, in case, the current value of the at least one vital sign exceeds the predetermined upper threshold and/or undercuts the lower threshold.

The occupant can be any person and/or animal, particularly the driver of the vehicle, or a passenger. The data transmitting connection can be a direct connection or an indirect connection. The direct connection defines a connection directly between the personal device and the on-board-device, e.g., via near field communication (NFC), via Bluetooth^{®}, etc., whereas an indirect connection defines a connection via internet, such as a 4G-network, a 5G-network and the like, and/or a cloud, e.g., via http-protocol, MQ Telemetry Transport (MGTT), gRPC etc.

The personal device can be a smart phone, a smart watch, a heart rate monitor, a blood sugar meter, etc., and the on-board-device is e.g., an infotainment head unit (IHU) of the vehicle. It is also possible that a vital monitor device, particularly medical devices, e.g., the blood sugar meter, is transmitting the collected or recorded vital signs, in this case blood sugar values, to another device, e.g., the smartphone, which is transmitting the values to the on-board-device. The vital sign is at least one of a pulse/heart rate, a blood pressure, a blood sugar, a rate of breathing, an oxygen saturation level, etc.

Furthermore, it is possible that values of two, three or more different types of vital signs are transmitted from the personal device to the on-board-device to be displayed simultaneously on the display device. In particular, the on-board-device receives personal data from the current value and from a predetermined past time interval, e.g., the past 24 hours, and this personal data is then stored in the storing unit. When new values are available, those new values are received by the on-board-device, e.g., by updating the data, and the oldest data will then be deleted, such that only data within the predetermined past time interval is stored in the storing unit.

The storing unit may be integrally formed in an electronic control unit (ECU), particularly of the on-board-device of the vehicle. Alternatively, the storing unit may be a unit being formed separate from the vehicle, e.g., a cloud. Setting the predetermined upper and/or lower threshold may be performed by a manual input, particularly from the occupant, e.g., via the display device. Alternatively, the predetermined upper and/or lower threshold may be set to values being received, particularly by the on-board-device of the vehicle, from an external device, such as the personal device, from an application (app) being installed on the personal device, from an external institution and/or person, e.g., a hospital, a medical center, a treating doctor etc.

The display device is a display device which is arranged in a passenger compartment of the vehicle, particularly in the driver's field of vision. For example, the display device is a display device being integrated in the vehicle, such as a center stack display. Alternatively, the display device may be an external device being connected to the on-board-device of the vehicle via a corresponding interface, e.g., a connecting port, such as a USB-port etc. Comparing the current value of the at least one vital sign with the predetermined upper and/or lower threshold may be performed by a processing unit being included in the vehicle, particularly in the on-board-device, but may alternatively be performed by an external unit, e.g., a program stored in a cloud, an external data processing unit, etc., which than transmits a comparison result to the on-board-device of the vehicle.

Notifying the occupant visually, e.g., by displaying a notification, e.g., an informational notification, on the display device is an easy and efficient way of notifying the occupant that the current value of the at least one vital sign is critical, namely has exceeded the predetermined upper threshold and/or has undercut the predetermined lower threshold. Notifying the occupant audibly, e.g., by an audio signal, an alarm tone etc., allows notifying the occupant that the current value of the at least one vital sign is critical, namely has exceeded the predetermined upper threshold and/or has undercut the predetermined lower threshold without the need of a display device. Notifying the occupant physically, e.g., by haptic feedback, such as a vibration of the steering wheel etc., allows notifying the occupant that the current value of the at least one vital sign is critical, namely has exceeded the predetermined upper threshold and/or has undercut the predetermined lower threshold, particularly in situations, in which an acoustic signal may not be recognized by the occupant, e.g., in case the occupant is listening to loud music and/or the occupant is deaf, etc. Further, it may be possible that a person other than the occupant/driver and/or an external institution, such as a predetermined emergency contact person, a treating doctor, a predetermined medical center etc., may be additionally notified.

The method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise one or more of a processor, a memory, a data interface, or the like.

Thus, the method allows quickly visualizing vital signs through a device in the occupant compartment of the vehicle, thereby increasing the awareness of vital sign values. Furthermore, the method provides aid accessibility options, particularly, in case of a detected critical vital sign value, thereby supporting the occupant, particularly the driver to react appropriately to the critical vital sign value. Thus, the method allows improving the safety of the occupant(s) in the vehicle, and possibly of the surroundings. In particular, the method allows mirroring personal data including at least one vital sign in the on-board-device. Thus, there is no need for including sensors for monitoring vital signs in the vehicle. In other words, the personal data used by the method is personal data collected and/or recorded by the personal device and being mirrored on the on-board-device of the vehicle.

According to an example, the method may further comprise a step of providing a notification to the occupant, the notification comprising at least one reaction-option for initiating a predetermined action based on the current value and/or type of the at least one vital sign, and an ignore-option for ignoring the notification.

The notification may also comprise two, three or more (re)action options, each comprising a different suggestion for reacting on the current value, which either exceeds the upper threshold or undercuts the lower threshold.

According to an example, the method may further comprise a step of initiating and/or performing the option being selected by the occupant.

In case, the occupant has selected one of the presented (re)action options, the corresponding predetermined (re)action may be initiated and/or performed. Alternatively, in case the occupant has selected the ignore-option, no (re)action may be initiated this time and the monitoring of the at least one vital sign may be continued. It may be also possible that, after the occupant has selected an option, the method may verify if the performance of the selected option is appropriate under current given circumstances, before initiating and/or performing the selected option.

According to an example, the notification may be provided to the occupant via displaying the notification on the display device. By this, the notification may be provided at least visually to the occupant, allowing the occupant to select quickly and easily one of the presented options.

According to an example, the part of the stored personal data being displayed on the display device may comprise a predetermined number of values of the at least one vital sign, the number being settable individually.

Displaying a predetermined number of values of the at least one vital sign, wherein the number may be particularly larger than one, may allow the driver recognizing a development or trend of the values of the at least one vital sign over time.

The method according to the first aspect comprises the step of updating the displayed values of the at least one vital sign continuously in predetermined intervals (e.g., every 5 to 10 seconds, every minute, every 5 minutes etc.).

By this, the displayed value(s) may always comprise the current value. A length or duration of the predetermined intervals may depend on the vital sign. For example, while monitoring a heart rate, updating the value(s) every 10 to 30 seconds may be appropriate, whereas while monitoring a blood sugar value, an interval length of 20 to 30 minutes may be sufficient for monitoring the blood sugar value. In other words, the predetermined interval may depend on a change rate of the respective monitored vital sign. For example, a change in the heart rate may become critical within a few second up to a few minutes, whereas a change in the blood sugar value is slower and therefore, may become critical within minutes to hours. The method according to the first aspect further comprises adapting the predetermined interval dependent on a development of the monitored vital sign values. For example, if a blood sugar value is monitored and a change of the value towards a critical value may be detected, the predetermined interval for updating the current value of the blood sugar may be shortened to be able to detect the current value exceeding the upper threshold or undercutting the lower threshold as early as possible.

According to an example, previous displayed values may be stored in the storing unit, particularly in addition to the values already stored in the storing unit. (e.g., for a predetermined amount of time (e.g., three hours, 5 hours, 12 hours, 24 hours etc., or a predetermined number of values).

In particular, the previous displayed values may be stored in the storing unit for a predetermined amount of time, e.g., all values that have been collected within the last one, two, three or more hours. Alternatively, a predetermined number of previously displayed values may be stored in the storing unit, e.g., the last ten, 20, 50, 100 values.

According to an example, the predetermined (re)action may be at least one of activating the automatic pilot, starting the navigation, particularly to a predetermined location, such as a gas station, grocery store, medical center, hospital etc., and initiating an emergency call, e.g., activating e-call etc.

For example, some vital sign values may indicate that the driver may not be feeling well, and therefore should not be driving the vehicle. In such case, one predetermined (re)action option being provided to the driver via the notification may comprise activating the automatic pilot, such as an autonomous driving mode. Other vital sign values may indicate that the driver may need food, e.g., in case of a critical blood sugar value, or medical help, e.g., in case of a critical heart rate and/or a critical blood pressure. In such cases, the predetermined (re)action option(s) may comprise starting the navigation, particularly to a predetermined location. Some vital sign values may even indicate that the driver may not be able to reach out for help himself. In such a case, one predetermined (re)action option may comprise initiating an emergency call, e.g., via activating the e-call function of the vehicle. Further, it may also be possible that, in case, the occupant does not select any of the options provided by the notification within a predetermined amount of time, the e-call function and/or the automatic pilot is activated automatically.

According to an example, all stored values of the at least one vital sign may be displayable, e.g., in form of a time-based diagram, on request of the occupant.

All values of a predetermined timeline may be helpful for medical staff in case, the driver needs medical help. Further, it may be helpful for the occupant/driver itself to see a long-term development of the monitored vital sign values for adapting the upper and/or lower threshold, and/or the length of interval for updating the vital sign value, thereby improving a detection of a critical vital sign value.

According to an example, the navigation may be performed using Google Android OS functions. Further, upon starting the navigation as the predetermined (re)action, an android intent may be created comprising the information according to the selected predetermined (re)action for starting Google Maps.

Google Android OS functions allows using the information of the surroundings of the vehicle, such as nearby patrol stations, grocery stores, medical centers, hospitals etc., the information being included in Google Maps.

This may improve the driver's perception of the notification. Further, it may alert occupants other than the driver of the notification and thus, may make them aware of a possible critical situation.

According to an example, the predetermined upper threshold and/or the predetermined lower threshold may be set by the occupant, e.g., by a manual input via the display device.

This may allow an individual setting of the thresholds. For example, a "normal" blood pressure may depend on the respective person. Some people have a higher blood pressure than other people, depending on the height and/or weight and/or (chronical) pre-existing conditions. Therefore, for detecting critical vital sign values, different thresholds may be required depending on the respective person.

The method according to the first aspect further comprises the step of deleting the personal data from the storing unit, when the connection between the personal device of the occupant and the on-board-device of the vehicle is terminated or disconnected, e.g., when the occupant leaves the car, or when the connection is disconnected by the occupant on purpose.

Thus, no personal data may be accessible to persons other than a current occupant, particularly the current driver, of the vehicle, thereby improving personal data security. This may be particularly advantageous in case of rental cars, shared cars, cars from a car pool etc.

According to an example, the at least one vital sign may be at least one of a pulse/heart rate, a blood pressure, a blood sugar, and a rate of breathing of the occupant.

According to a second aspect, there is provided a system for monitoring vital data of a occupant, particularly a driver, of a vehicle. The system comprises a receiving device, a storing unit, a setting unit, a display device, a comparing unit, and a notification unit.

The receiving device is configured to receive personal data comprising at least one vital sign from a personal device via the data transmitting connection, the personal device being configured to collect and/or record the personal data comprising the at least one vital sign of the user/owner, particularly the occupant. The data transmitting connection may be a direct or indirect data transmitting connection being established between the personal device and an on-board-device of the vehicle, e.g., by a connecting device. The storing unit is configured to store the personal data and configured to delete the personal data from the storing unit, when the connection between the personal device of the occupant and the on-board-device of the vehicle is terminated. The storing unit may be integrally formed in an electronic control unit (ECU), particularly of the on-board-device of the vehicle, and thus may be configured to store the personal data locally. Alternatively, the storing unit may be a unit being formed separate from the vehicle, e.g., a cloud.

The setting unit is configured to set at least one of a predetermined upper threshold and a predetermined lower threshold for the at least one vital sign. Therefore, the setting unit may be configured to receive the predetermined upper and/or lower threshold being manually input, particularly from the occupant, e.g., via the display device. Alternatively, the predetermined upper and/or lower threshold may be set to values being received from an external device, such as the personal device, from an application (app) being installed on the personal device, from an external institution and/or person, e.g., a hospital, a medical center, a treating doctor etc. The display device is configured to display at least a part of the stored personal data, the predetermined upper threshold and/or the predetermined lower threshold for the at least one vital sign, wherein the part of the stored personal data comprises at least a current value of the at least one vital sign. The display device is further configured to update the displayed values of the at least one vital sign continuously in predetermined intervals, wherein the predetermined intervals are adapted dependent on a development of the values of the at least one vital sign. The display device is a display device which is arranged in a passenger compartment of the vehicle, particularly in the driver's field of vision. For example, the display device is a display device being integrated in the vehicle, such as a center stack display. Alternatively, the display device is an external device being connected to the on-board-device of the vehicle via a corresponding interface, e.g., a connecting port, such as a USB-port etc.

The comparing unit is configured to compare the current value of the at least one vital sign with the predetermined upper and/or lower threshold. For example, the comparing unit can be integrally formed in the vehicle, particularly in the on-board-device. Alternatively, the comparing unit can be an external unit, e.g., a program stored in a cloud, an external data processing unit, etc., which than transmits a comparison result to the on-board-device of the vehicle.

The notification unit is configured to notify the occupant visually and/or audibly and/or physically in case, the current value of the at least one vital sign exceeds the predetermined upper threshold and/or undercuts, that means falls below, the lower threshold. Further, the notification device may additionally be configured to notify a person other than the occupant/driver and/or an institution, such as a predetermined emergency contact person, a treating doctor, a predetermined medical center etc.

The direct connection defines a connection directly between the personal device and the on-board-device, e.g., via near field communication (NFC), via Bluetooth^{®}, etc., whereas an indirect connection defines a connection via internet, such as a 4G-network, a 5G-network and the like, and/or a cloud, e.g., via http-protocol, MQ Telemetry Transport (MGTT), gRPC etc.

The personal device can be a smart phone, a smart watch, a heart rate monitor, a blood sugar meter, etc., and the on-board-device is e.g., an infotainment head unit (IHU) of the vehicle. It is also possible that vital monitor device, particularly medical devices, e.g., the blood sugar meter, is transmitting the collected or recorded vital signs, in this case blood sugar values, to another device, e.g., the smartphone, which is transmitting the values to the on-board-device. The vital sign is at least one of a pulse/heart rate, a blood pressure, a blood sugar, a rate of breathing, an oxygen saturation level, etc.

The storing unit can be integrally formed in an electronic control unit (ECU), particularly of the on-board-device of the vehicle. The display device is a display device which is arranged in an occupant compartment of the vehicle, particularly in the driver's field of vision. For example, the display device is a center stack display.

Thus, the system allows quickly visualizing vital signs through a device in the occupant compartment of the vehicle, thereby increasing the awareness of vital sign values. Furthermore, the system provides aid accessibility options, particularly, in case of a detected critical vital sign value, thereby supporting the occupant, particularly the driver to react appropriately to the critical vital sign value. Thus, the system allows improving the safety of the occupant(s) in the vehicle, and possibly of the surroundings. In particular, the system enables mirroring personal data including at least one vital sign being collected from an external (personal device) in the on-board-device. Thus, there is no need for including sensors for monitoring vital signs in the vehicle.

According to an example, the notification unit may be further configured to provide a notification to the occupant, the notification comprising at least one reaction-option for initiating a predetermined action based on the current value and/or type of the at least one vital sign and an ignore-option for ignoring the notification.

The notification can also comprise two, three or more (re)action options, each comprising a different suggestion for reacting on the current value, which either exceeds the upper threshold or undercuts the lower threshold.

According to an example, the system may further comprise a processing unit being configured to initiate and/or perform the option selected by the occupant.

In case, the occupant has selected one of the presented (re)action options, the corresponding predetermined (re)action is initiated. Alternatively, in case the occupant has selected the ignore-option, no (re)action is initiated this time and the monitoring of the at least one vital sign is continued. It may be also possible that, after the occupant has selected an option, the processing unit may be configured to verify if the performance of the selected option is appropriate under current given circumstances, before initiating and/or performing the selected option.

According to an example, the connecting device and/or the receiving device and/or the storing unit and/or the setting unit and/or the comparing unit and/or the notification unit and/or the processing unit may be integrally formed with an electronic control unit (ECU) of the on-board-device of the vehicle.

According to an example, the notification unit may be configured to provide the notification to the occupant via displaying the notification on the display device.

This may allow the occupant to quickly and easily select one of the presented options.

According to an example, the setting unit may be configured to receive a manual input of the occupant, e.g., via the display device, the input being used to define/set the predetermined upper threshold and/or the predetermined lower threshold.

This may allow an individual setting of the thresholds. For example, a "normal" blood pressure may depend on the respective person. Some people have a higher blood pressure than other people, depending on the height and/or weight and/or (chronical) pre-existing conditions. Therefore, for detecting critical vital sign values, different thresholds may be required depending on the respective person.

According to an example, the system may be configured to perform a method according to the first aspect.

It should be noted that the above examples may be combined with each other irrespective of the aspect involved. Accordingly, the method may be combined with structural features and, likewise, the apparatus and the system may be combined with features described above with regard to the method.

These and other aspects of the present disclosure will become apparent from and elucidated with reference to the embodiments described hereinafter.

### BRIEF DESCRIPTION OF DRAWINGS

Exemplary examples of the disclosure will be described in the following with reference to the following drawings.
- Fig. 1: shows a schematic flow diagram of an exemplary method for monitoring vital data of an occupant of a vehicle according to the present disclosure.
- Fig. 2A: shows a schematic diagram exemplarily illustrating a direct connection according to the present disclosure.
- Fig. 2B: shows a schematic diagram exemplarily illustrating an indirect connection according to the present disclosure.
- Fig. 3: show a schematic block diagram of an exemplary system for monitoring vital data of an occupant of a vehicle according to the present disclosure.

The figures are merely schematic representations and serve only to illustrate examples of the disclosure. Identical or equivalent elements are in principle provided with the same reference signs.

### DESCRIPTION OF EMBODIMENTS

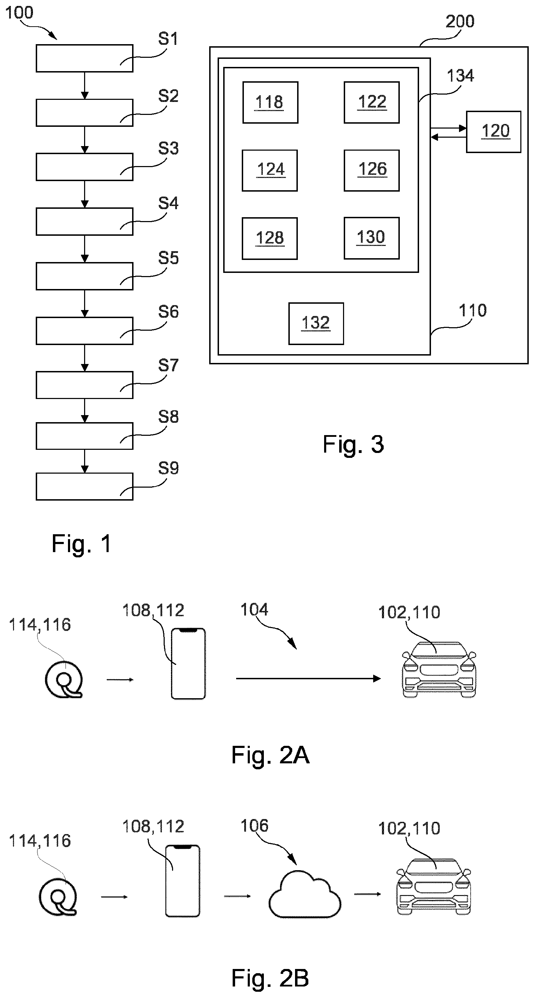

Figure 1 shows schematically and exemplarily a flow diagram of an example of a method 100 for monitoring vital data of an occupant of a vehicle 102 (see Figures 2A and 2B). The exemplary method 100 comprises the following steps, which are not necessarily performed in the shown order.

In a step S1, a direct connection 104 (see also Figure 2A) or an indirect connection 106 (see also Figure 2B) between a personal device 108 (see also Figures 2A and 2B) of the occupant and an on-board-device 110 (see also Figure 3) of the vehicle 102 is established, the personal device 108 being configured to collect and/or record personal data comprising at least one vital sign.

The personal device 108 in Figures 2A and 2B is exemplarily shown as a smartphone 112, but may also be a smart watch, a heart rate monitor, a blood sugar meter, an oximeter etc. Further, the personal device 108 may be configured to collect and/or record personal data itself or to receive collected and/or recorded data from an external device 114, particularly a medical device, in Figured 2A and 2B exemplarily shown as a blood sugar meter 116. vital sign or vital data may be a pulse, a blood pressure, a blood sugar, a rate of breathing, an oxygen saturation level etc. The on-board-device 110 will be described below with regard to Figure 3.

The direct connection 104, as exemplarily shown in Figure 2A, is defined as a connection being established directly between the personal device 108 and the on-board-device 110, e.g., via near field communication (NFC), via Bluetooth^{®}, etc. The indirect connection 106, as exemplarily shown in Figure 2B, is defined as a connection via internet, such as a 4G-network, a 5G-network end the like, and/or a cloud, e.g., via http-protocol, etc.

In a step S2, the on-board-device 110 of the vehicle 102 receives the personal data comprising the at least one vital sign from the personal device 108 via the data transmitting connection 104, 106. In a step S3, the received personal data is stored locally in a storing unit 118 (see Figure 3) of the on-board device 110 of the vehicle 102. In a step S4, at least one of a predetermined upper threshold and a predetermined lower threshold for the at least one vital sign is/are set. The setting may be particularly performed manually by a user, e.g., the occupant, whose personal data is transmitted to the on-board-device 110, particularly the driver.

In a step S5, at least a part of the personal data stored by the storing unit 118, the predetermined upper threshold and/or the predetermined lower threshold for the at least one vital sign, are displayed on a display device 120 (see Figure 3), e.g., a center stack display, of the vehicle 102, wherein the part of the stored personal data comprises at least a current value of the at least one vital sign. In a step S6, the current value of the at least one vital sign is compared with the predetermined upper threshold and/or the predetermined lower threshold, to determine whether the current value of the at least one vital sign exceeds the predetermined upper threshold or undercuts the predetermines lower threshold.

In a step S7, the occupant is notified, in case, the current value of the at least one vital sign exceeds the predetermined upper threshold and/or undercuts the lower threshold, and in a step S8, a notification is provided to the occupant, the notification comprising at least one reaction-option for initiating a predetermined (re)action based on the current value and/or a type of the at least one vital sign, and an ignore-option for ignoring the notification. In a step S9, the option being selected by the occupant from the notification is performed.

Figure 3 shows schematically and exemplarily a system 200 for monitoring vital data of an occupant of a vehicle 102. The system 200 comprises a connecting device 122, a receiving device 124, the storing unit 118, a setting unit 126, the display device 120, a comparing unit 128, a notification unit 130, and a processing unit 132.

The connecting device 122 may be, for example, a Bluetooth^{®} interface or an internet-enabling device, and is configured to establish either the direct connection 104 or the indirect connection 106 between the personal device 108 and the on-board-device 110. The receiving device 124, e.g., a receiver, is configured to receive the personal data comprising at least one vital sign from the person device 108 via the data transmitting connection 104, 106. The storing unit 118 is configured to store the personal data locally, and the setting unit 126 is configured to set at least one of a predetermined upper threshold and a predetermined lower threshold for the at least one vital sign.

The display device 120 may be a center stack display or any other display being arranged in the driver's visual field, and is configured to display at least a part of the stored personal data, the predetermined upper threshold and/or the predetermined lower threshold for the at least one vital sign, wherein the part of the stored personal data comprises at least a current value of the at least one vital sign. The comparing unit 128 is configured to compare the current value of the at least one vital sign with the predetermined upper threshold and/or the predetermined lower threshold, and the notification unit 130 is configured to issue displaying a notification on the display device 120 for notifying the occupant, in case, the current value of the at least one vital sign exceeds the predetermined upper threshold or undercuts the lower threshold. The notification is configured to provide the occupant at least one reaction-option for initiating a predetermined (re)action based on the current value and/or a type) of the at least one vital sign, and an ignore-option for ignoring the notification.

The processing unit 132 is configured to perform the option being selected by the occupant, e.g., initiating the predetermined (re)action, in case the occupant has selected the at least one reaction-option, or terminating the process, in case the occupant selects the ignore-option. As exemplarily shown in Figure 3, the storing unit 118, the connecting device 122, the receiving device 124, the setting unit 126, the comparing unit 128, and the notification unit 130 are integrally formed in an electronic control unit (ECU) 134 of the on-board-device 110 of the vehicle 102. Further, not shown in the Figures, also the processing unit 132 may be integrally formed with the ECU 134 of the on-board-device 110.

Other variations to the disclosed examples can be understood and effected by those skilled in the art in practicing the claimed disclosure, from the study of the drawings, the disclosure, and the appended claims. In the claims the word "comprising" does not exclude other elements or steps and the indefinite article "a" or "an" does not exclude a plurality. A single processor or other unit may fulfill the functions of several items or steps recited in the claims. The mere fact that certain measures are recited in mutually different dependent claims does not indicate that a combination of these measures cannot be used to advantage. A computer program may be stored/distributed on a suitable medium such as an optical storage medium or a solid-state medium supplied together with or as part of other hardware, but may also be distributed in other forms, such as via the Internet or other wired or wireless telecommunication systems. Any reference signs in the claims should not be construed as limiting the scope of the claims.

### LIST OF REFERENCE SIGNS

- 100: method
- 102: vehicle
- 104: direct connection
- 106: indirect connection
- 108: personal device
- 110: on-board-device
- 112: smartphone
- 114: external device
- 116: blood sugar meter
- 118: storing unit
- 120: display device
- 122: connecting device
- 124: receiving device
- 126: setting unit
- 128: comparing unit
- 130: notification unit
- 132: processing unit
- 134: electronic control unit (ECU)
- 200: system

## Claims

1. A method (100) for monitoring vital data of an occupant of a vehicle (102),
the method (100) comprising:
receiving, by an on-board-device (110) of the vehicle (102), personal data comprising at least one vital sign from a personal device (108) of the occupant, the personal device being configured to collect and/or record the personal data comprising the at least one vital sign,
storing the personal data in a storing unit (118),
setting at least one of a predetermined upper threshold and a predetermined lower threshold for the at least one vital sign;
displaying at least a part of the stored personal data on a display device (120), the predetermined upper threshold and/or the predetermined lower threshold for the at least one vital sign, wherein the part of the stored personal data comprises at least a current value of the at least one vital sign;
updating the displayed values of the at least one vital sign continuously in predetermined intervals, wherein the predetermined intervals are adapted dependent on a development of the values of the at least one vital sign;
comparing the current value of the at least one vital sign with the predetermined upper threshold and/or the predetermined lower threshold;
notifying the occupant visually and/or audibly and/or physically, in case, the current value of the at least one vital sign exceeds the predetermined upper threshold and/or undercuts the lower threshold; and
deleting the personal data from the storing unit (118), when a connection (104, 106) between the personal device (108) of the occupant and the on-board-device (110) of the vehicle (102) is terminated.

2. The method (100) according to claim 1, further comprising providing a notification to the occupant, the notification comprising at least one reaction-option for initiating a predetermined action based on the current value of the at least one vital sign, and an ignore-option for ignoring the notification.

3. The method according to claim 2, further comprising a step of initiating and/or performing the option being selected by the occupant.

4. The method (100) according to any of claims 1 to 3, wherein the part of the stored personal data being displayed on the display device (120) comprises a predetermined number of values of the at least one vital sign, the number being settable individually.

5. The method (100) according to any of claims 1 to 4, wherein the predetermined action is at least one of activating the automatic pilot, starting the navigation, particularly to a predetermined location and initiating an emergency call.

6. The method (100) according to any of claims 1 to 5, wherein all stored values of the at least one vital sign are displayable on request of the occupant.

7. The method (100) according to any of claims 1 to 6, wherein the predetermined upper threshold and/or the predetermined lower threshold are set by the occupant.

8. The method (100) according to any of claims 1 to 7, wherein the at least one vital sign is at least one of a pulse, a blood pressure, a blood sugar, and a rate of breathing of the occupant.

9. A system (200) for monitoring vital data of an occupant of a vehicle (102), the system (200) comprising:
a receiving device (124) configured to receive personal data comprising at least one vital sign from a personal device (108) of the occupant, the personal device (108) being configured to collect and/or record the personal data comprising the at least one vital sign,
a storing unit (118) configured to store the personal data,
the storing unit (118) being further configured to delete the personal data from the storing unit (118), when a
connection (104, 106) between the personal device (108) of the occupant and an on-board-device (110) of the vehicle (102) is terminated;
a setting unit (126) configured to set at least one of a predetermined upper threshold and a predetermined lower threshold for the at least one vital sign,
a display device (120) configured to display at least a part of the stored personal data, the predetermined upper threshold and/or the predetermined lower threshold for the at least one vital sign, wherein the part of the stored personal data comprises at least a current value of the at least one vital sign;
the display device (120) being further configured to update the displayed values of the at least one vital sign continuously in predetermined intervals, wherein the predetermined intervals are adapted dependent on a development of the values of the at least one vital sign;
a comparing unit (128) configured to compare the current value of the at least one vital sign with the predetermined upper threshold and/or the predetermined lower threshold;
a notification unit (130) configured to notify the occupant visually and/or audibly and/or physically, in case, the current value of the at least one vital sign exceeds the predetermined upper threshold and/or undercuts the lower threshold.

10. The system (200) according to claim 9, the notification unit (130) being further configured to provide a notification to the occupant, the notification comprising at least one reaction-option for initiating a predetermined action based on the current value of the at least one vital sign and an ignore-option for ignoring the notification.

11. The system (200) according to claim 10, further comprising a processing unit (132), the processing unit (132) being configured to initiate and/or perform the option selected by the occupant.

12. The system (200) according to any of claims 9 to 11, the setting unit (126) being configured to receive a manual input of the occupant, the input being used to set the predetermined upper threshold and/or the predetermined lower threshold.

13. The system (200) according to any of claims 9 to 12 being configured to perform a method (100) according any of claims 1 to 8.

## Patentansprüche

1. Ein Verfahren (100) zum Überwachen von Vitaldaten eines Insassen eines Fahrzeugs (102),
wobei das Verfahren (100) Folgendes umfasst:
Empfangen von persönlichen Daten, die wenigstens ein Vitalzeichen umfassen, von einem persönlichen Gerät (108) des Insassen durch ein Bordgerät (110) des Fahrzeugs (102), wobei das persönliche Gerät dazu ausgestaltet ist, die persönlichen Daten, die das wenigstens eine Vitalzeichen umfassen, zu sammeln und/oder aufzuzeichnen,
Speichern der persönlichen Daten in einer Speichereinheit (118),
Einstellen wenigstens eines aus einem vorbestimmten oberen Schwellwert und einem vorbestimmten unteren Schwellwert für das wenigstens eine Vitalzeichen;
Anzeigen wenigstens eines Teils der gespeicherten persönlichen Daten auf einer Anzeigevorrichtung (120), des vorbestimmten oberen Schwellwerts und/oder des vorbestimmten unteren Schwellwerts für das wenigstens eine Vitalzeichen, wobei der Teil der gespeicherten persönlichen Daten wenigstens einen aktuellen Wert des wenigstens einen Vitalzeichens umfasst;
Aktualisieren der angezeigten Werte des wenigstens einen Vitalzeichens kontinuierlich in vorbestimmten Intervallen, wobei die vorbestimmten Intervalle in Abhängigkeit von einer Entwicklung der Werte des wenigstens einen Vitalzeichens angepasst werden;
Vergleichen des aktuellen Werts des wenigstens einen Vitalzeichens mit dem vorbestimmten oberen Schwellwert und/oder dem vorbestimmten unteren Schwellwert;
visuelles und/oder akustisches und/oder physisches Benachrichtigen des Insassen, falls der aktuelle Wert des wenigstens einen Vitalzeichens den vorbestimmten oberen Schwellwert überschreitet und/oder den unteren Schwellwert unterschreitet; und
Löschen der persönlichen Daten aus der Speichereinheit (118), wenn eine Verbindung (104, 106) zwischen dem persönlichen Gerät (108) des Insassen und dem Bordgerät (110) des Fahrzeugs (102) beendet wird.

2. Das Verfahren (100) gemäß Anspruch 1, ferner umfassend das Bereitstellen einer Benachrichtigung an den Insassen, wobei die Benachrichtigung wenigstens eine Reaktionsoption zum Einleiten einer vorbestimmten Aktion basierend auf dem aktuellen Wert des wenigstens einen Vitalzeichens und eine Ignorieroption zum Ignorieren der Benachrichtigung umfasst.

3. Das Verfahren gemäß Anspruch 2, ferner umfassend einen Schritt zum Einleiten und/oder Ausführen der vom Insassen ausgewählten Option.

4. Das Verfahren (100) gemäß einem der Ansprüche 1 bis 3, wobei der auf der Anzeigevorrichtung (120) angezeigte Teil der gespeicherten persönlichen Daten eine vorbestimmte Anzahl von Werten des wenigstens einen Vitalzeichens umfasst, wobei die Anzahl individuell einstellbar ist.

5. Das Verfahren (100) gemäß einem der Ansprüche 1 bis 4, wobei die vorbestimmte Aktion wenigstens eine der folgenden ist: Aktivieren des Autopiloten, Starten der Navigation, insbesondere zu einem vorbestimmten Ort, und Auslösen eines Notrufs.

6. Das Verfahren (100) gemäß einem der Ansprüche 1 bis 5, wobei alle gespeicherten Werte des wenigstens einen Vitalzeichens auf Anfrage des Insassen anzeigbar sind.

7. Das Verfahren (100) gemäß einem der Ansprüche 1 bis 6, wobei der vorbestimmte obere Schwellwert und/oder der vorbestimmte untere Schwellwert vom Insassen eingestellt werden.

8. Das Verfahren (100) gemäß einem der Ansprüche 1 bis 7, wobei das wenigstens eine Vitalzeichen wenigstens eines der folgenden ist: Puls, Blutdruck, Blutzucker und Atemfrequenz des Insassen.

9. Ein System (200) zum Überwachen von Vitaldaten eines Insassen eines Fahrzeugs (102), wobei das System (200) Folgendes umfasst:
ein Empfangsgerät (124), das dazu ausgestaltet ist, persönliche Daten, die wenigstens ein Vitalzeichen umfassen, von einem persönlichen Gerät (108) des Insassen zu empfangen, wobei das persönliche Gerät (108) dazu ausgestaltet ist, die persönlichen Daten, die das wenigstens eine Vitalzeichen umfassen, zu sammeln und/oder aufzuzeichnen,
eine Speichereinheit (118), die zum Speichern der persönlichen Daten ausgestaltet ist,
wobei die Speichereinheit (118) ferner dazu ausgestaltet ist, die persönlichen Daten aus der Speichereinheit (118) zu löschen, wenn eine Verbindung (104, 106) zwischen dem persönlichen Gerät (108) des Insassen und einem Bordgerät (110) des Fahrzeugs (102) beendet wird;
eine Einstelleinheit (126), die dazu ausgestaltet ist, für das wenigstens eine Vitalzeichen wenigstens einen aus einem vorbestimmten oberen Schwellwert und einem vorbestimmten unteren Schwellwert einzustellen,
eine Anzeigevorrichtung (120), die dazu ausgestaltet ist, zumindest einen Teil der gespeicherten persönlichen Daten, den vorbestimmten oberen Schwellwert und/oder den vorbestimmten unteren Schwellwert für das wenigstens eine Vitalzeichen anzuzeigen, wobei der Teil der gespeicherten persönlichen Daten wenigstens einen aktuellen Wert des wenigstens einen Vitalzeichens umfasst;
wobei die Anzeigevorrichtung ferner dazu ausgestaltet ist, die angezeigten Werte des wenigstens einen Vitalzeichens kontinuierlich in vorbestimmten Intervallen zu aktualisieren, wobei die vorbestimmten Intervalle in Abhängigkeit von einer Entwicklung der Werte des wenigstens einen Vitalzeichens angepasst werden;
eine Vergleichseinheit (128), die dazu ausgestaltet ist, den aktuellen Wert des wenigstens einen Vitalzeichens mit dem vorbestimmten oberen Schwellwert und/oder dem vorbestimmten unteren Schwellwert zu vergleichen;
eine Benachrichtigungseinheit (130), die dazu ausgestaltet ist, den Insassen optisch und/oder akustisch und/oder physisch zu benachrichtigen, falls der aktuelle Wert des wenigstens einen Vitalzeichens den vorbestimmten oberen Schwellwert überschreitet und/oder den unteren Schwellwert unterschreitet.

10. Das System (200) gemäß Anspruch 9, wobei die Benachrichtigungseinheit (130) ferner dazu ausgestaltet ist, dem Insassen eine Benachrichtigung bereitzustellen, wobei die Benachrichtigung wenigstens eine Reaktionsoption zum Einleiten einer vorbestimmten Aktion basierend auf dem aktuellen Wert des wenigstens einen Vitalzeichens und eine Ignorieroption zum Ignorieren der Benachrichtigung umfasst.

11. Das System (200) gemäß Anspruch 10, ferner umfassend eine Verarbeitungseinheit (132), wobei die Verarbeitungseinheit (132) dazu ausgestaltet ist, die vom Insassen ausgewählte Option einzuleiten und/oder auszuführen.

12. Das System (200) gemäß einem der Ansprüche 9 bis 11, wobei die Einstelleinheit (126) dazu ausgestaltet ist, eine manuelle Eingabe des Insassen zu empfangen, wobei die Eingabe dazu verwendet wird, den vorbestimmten oberen Schwellwert und/oder den vorbestimmten unteren Schwellwert einzustellen.

13. Das System (200) gemäß einem der Ansprüche 9 bis 12, wobei das System (200) so ausgestaltet ist, dass es ein Verfahren (100) gemäß einem der Ansprüche 1 bis 8 ausführt.

## Revendications

1. Procédé (100) de surveillance de données vitales d'un occupant d'un véhicule (102), le procédé (100) comprenant :
la réception, par un dispositif embarqué (110) du véhicule (102), de données personnelles comprenant au moins un signe vital provenant d'un dispositif personnel (108) de l'occupant, le dispositif personnel étant configuré pour collecter et/ou enregistrer les données personnelles comprenant l'au moins un signe vital,
le stockage des données personnelles dans une unité de stockage (118),
le réglage d'au moins un parmi un seuil supérieur prédéterminé et un seuil inférieur prédéterminé pour l'au moins un signe vital ;
l'affichage d'au moins une partie des données personnelles stockées sur un dispositif d'affichage (120), du seuil supérieur prédéterminé et/ou du seuil inférieur prédéterminé pour l'au moins un signe vital, dans lequel la partie des données personnelles stockées comprend au moins une valeur actuelle de l'au moins un signe vital ;
la mise à jour des valeurs affichées de l'au moins un signe vital en continu à intervalles prédéterminés, dans lequel les intervalles prédéterminés sont adaptés en fonction d'un développement des valeurs de l'au moins un signe vital ;
la comparaison de la valeur actuelle de l'au moins un signe vital avec le seuil supérieur prédéterminé et/ou le seuil inférieur prédéterminé ;
la notification à l'occupant visuellement et/ou par un signal sonore et/ou physiquement, si la valeur actuelle de l'au moins un signe vital dépasse le seuil supérieur prédéterminé et/ou descend en dessous du seuil inférieur ; et
la suppression des données personnelles de l'unité de stockage (118), lorsqu'une connexion (104, 106) entre le dispositif personnel (108) de l'occupant et le dispositif embarqué (110) du véhicule (102) est terminée.

2. Procédé (100) selon la revendication 1, comprenant en outre la fourniture d'une notification à l'occupant, la notification comprenant au moins une option « réaction » pour lancer une action prédéterminée basée sur la valeur actuelle de l'au moins un signe vital, et une option « ignorer » pour ignorer la notification.

3. Procédé selon la revendication 2, comprenant en outre une étape consistant à lancer et/ou à réaliser l'option sélectionnée par l'occupant.

4. Procédé (100) selon l'une quelconque des revendications 1 à 3, dans lequel la partie des données personnelles stockées affichées sur le dispositif d'affichage (120) comprend un nombre prédéterminé de valeurs de l'au moins un signe vital, le nombre étant réglable individuellement.

5. Procédé (100) selon l'une quelconque des revendications 1 à 4, dans lequel l'action prédéterminée est au moins une action consistant à activer le pilotage automatique, démarrer la navigation, en particulier jusqu'à un endroit particulier et lancer un appel d'urgence.

6. Procédé (100) selon l'une quelconque des revendications 1 à 5, dans lequel toutes les valeurs stockées de l'au moins un signe vital peuvent être affichées sur demande de l'occupant.

7. Procédé (100) selon l'une quelconque des revendications 1 à 6, dans lequel le seuil supérieur prédéterminé et/ou le seuil inférieur prédéterminé sont réglés par l'occupant.

8. Procédé (100) selon l'une quelconque des revendications 1 à 7, dans lequel l'au moins un signe vital est au moins un parmi un pouls, une pression artérielle, une glycémie, et une fréquence respiratoire de l'occupant.

9. Système (200) de surveillance de données vitales d'un occupant d'un véhicule (102), le système (200) comprenant :
un dispositif de réception (124) configuré pour recevoir des données personnelles comprenant au moins un signe vital d'un dispositif personnel (108) de l'occupant, le dispositif personnel (108) étant configuré pour collecter et/ou enregistrer les données personnelles comprenant l'au moins un signe vital,
une unité de stockage (118) configurée pour stocker les données personnelles,
l'unité de stockage (118) étant en outre configurée pour supprimer les données personnelles de l'unité de stockage (118), lorsqu'une connexion (104, 106) entre le dispositif personnel (108) de l'occupant et un dispositif embarqué (110) du véhicule (102) est terminée ;
une unité de réglage (126) configurée pour régler au moins un parmi un seuil supérieur prédéterminé et un seuil inférieur prédéterminé pour l'au moins un signe vital,
un dispositif d'affichage (120) configuré pour afficher au moins une partie des données personnelles stockées, le seuil supérieur prédéterminé et/ou le seuil inférieur prédéterminé pour l'au moins un signe vital, dans lequel la partie des données personnelles stockées comprend au moins une valeur actuelle de l'au moins un signe vital ;
le dispositif d'affichage (120) étant en outre configuré pour mettre à jour les valeurs affichées de l'au moins un signe vital en continu à des intervalles prédéterminés, dans lequel les intervalles prédéterminés sont adaptés en fonction d'un développement des valeurs de l'au moins un signe vital ;
une unité de comparaison (128) configurée pour comparer la valeur actuelle de l'au moins un signe vital avec le seuil supérieur prédéterminé et/ou le seuil inférieur prédéterminé ;
une unité de notification (130) configurée pour informer l'occupant visuellement et/ou par un signal sonore et/ou physiquement, si la valeur actuelle de l'au moins un signe vital dépasse le seuil supérieur prédéterminé et/ou est inférieure au seuil inférieur prédéterminé.

10. Système (200) selon la revendication 9, l'unité de notification (130) étant en outre configurée pour fournir une notification à l'occupant, la notification comprenant au moins une option « réaction » pour lancer une action prédéterminée sur la base de la valeur actuelle de l'au moins un signe vital et une option « ignorer » pour ignorer la notification.

11. Système (200) selon la revendication 10, comprenant en outre une unité de traitement (132), l'unité de traitement (132) étant configurée pour lancer et/ou effectuer l'option sélectionnée par l'occupant.

12. Système (200) selon l'une quelconque des revendications 9 à 11, l'unité de réglage (126) étant configurée pour recevoir une entrée manuelle de l'occupant, l'entrée étant utilisée pour régler le seuil supérieur prédéterminé et/ou le seuil inférieur prédéterminé.

13. Système (200) selon l'une quelconque des revendications 9 à 12, configuré pour metre en œuvre un procédé (100) selon l'une quelconque des revendications 1 à 8.
